# EUROPÄISCHE PATENTANMELDUNG

(11) **EP 0 523 556 A1**
(43) Veröffentlichungstag der Anmeldung: **20.01.1993**
(21) Anmeldenummer: 92111742.0
(22) Anmeldetag: 10.07.1992
(51) Int. Cl.: C07D 207/448, C07D 207/452

(54) **Verfahren zur Herstellung von N-tert.-Butoxycarbonyl-maleimid**

(30) Priorität: 19.07.1991 DE 4124028
(71) Anmelder: HOECHST AKTIENGESELLSCHAFT, D-65926 Frankfurt am Main (DE)
(72) Erfinder: Röschert, Horst, Dr., W-6531 Ober Hilbersheim (DE); Dammel, Ralph, Dr., Coventry, Rhode Island 02816 (US); Pawlowski, Georg, Dr., W-6200 Wiesbaden (DE); Przybilla, Klaus-Jürgen, Dr., W-6000 Frankfurt am Main 1 (DE)

(57) **Zusammenfassung**

Verfahren zur Herstellung von N-tert.-Butoxycarbonylmaleimid aus Maleimid und Di-tert.-butyl-dicarbonat, das dadurch gekennzeichnet ist, daß das Maleimid mit dem Di-tert.-butyl-dicarbonat in Gegenwart einer basischen Verbindung der allgemeinen Formel I 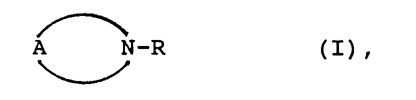
worin A die zur Vervollständigung eines fünf- oder sechsgliedrigen gesättigten Ringes erforderlichen Ringglieder bezeichnet und R für (C₁-C₄)Alkyl steht,in einer Stufe zu dem Endprodukt umgesetzt wird. Bei der Verbindung der allgemeinen Formel I handelt es sich insbesondere um N-Methyl-morpholin.

## Beschreibung

Die Erfindung betrifft ein Verfahren zur Herstellung von N-tert.-Butoxycarbonyl-maleimid aus Maleimid und Di-tert.-butyl-dicarbonat.

N-tert.-Butoxycarbonyl-maleimid und Copolymere davon mit Styrol wurden auf dem "Fall National Meeting, 13.-14. Okt. 1989" der Polymer Society of Korea referiert (s. Abstract of Papers, S. 56/57). Homo- und Copolymere mit t-BOC-Maleimid-Einheiten sind auch in der US-A 4 775 609 und der EP-A 0 234 327 offenbart. Hergestellt sind sie aus den entsprechenden Polymeren ohne t-BOC-Gruppen. Über Verfahren zur Herstellung von monomerem t-BOC-Maleimid wurde an diesen Stellen nichts bekannt.

tert.-Butylcarbamate sowie deren Herstellung aus sekundären Aminen und Derivaten der Kohlensäure sind bekannt (T. W. Greene, Protective Groups in Organic Synthesis, S. 232, New York 1981). Die Einführung der tert.-Butoxycarbonyl("t-BOC")-Schutzgruppe in Maleimid war bisher mit großen Schwierigkeiten verbunden. Wurde die Umsetzung unter basischen Bedingungen versucht, beispielsweise unter Verwendung von Alkali (z. B. Kaliumcarbonat), Trialkylaminen (z. B. Triethylamin oder Pyridin), Metallhydriden (z. B. NaH), Alkoholaten (z. B. Kalium-tert.-butanolat) oder lithiumorganischen Verbindungen (z. B. tert.-Butyl-lithium), so trat meistens eine Polymerisation des Maleimids ein. Saure Bedingungen erwiesen sich ebenfalls als ungeeignet, da die t-BOC-Gruppe durch Säure wieder abgespalten wird. In einem sauren Medium stellt sich daher bestenfalls ein dynamisches Gleichgewicht zwischen freiem und t-BOC-geschütztem Amin ein.

Durch eine mehrstufige Verfahrensweise läßt sich die in basischen Medien allgemein beobachtete Polymerisation verhindern. Dazu wird zunächst die C-C-Doppelbindung des Maleimids "geschützt", z. B. durch eine Diels-Alder-Reaktion mit Cyclopentadien. In einer Folgestufe kann dann die Umsetzung mit Di-tert.-butyl-dicarbonat in einem basischen Medium erfolgen. Anschließend muß die Doppelbindung wieder eingeführt werden, z.B. durch eine Retro-Diels-Alder-Reaktion. Ein solch aufwendiges, mehrstufiges Verfahren ist jedoch unter praktischen Aspekten wenig aussichtsreich.

Es bestand daher die Aufgabe, ein einstufiges Verfahren zur Herstellung von t-BOC-Maleimid zur Verfügung zu stellen.

Gemäß der vorliegenden Erfindung wird diese Aufgabe gelöst durch Umsetzung von Maleimid mit Di-tert.-butyl-dicarbonat in Gegenwart von N-Methyl-morpholin.

Gegenstand der Erfindung ist somit ein Verfahren zur Herstellung von N-tert.-butoxycarbonyl-maleimid aus Maleimid und Di-tert.-butyl-dicarbonat, das dadurch gekennzeichnet ist, daß das Maleimid mit dem Di-tert.-butyl-dicarbonat in Gegenwart einer basischen Verbindung der allgemeinen Formel I 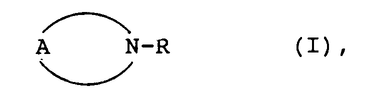
worin A die zur Vervollständigung eines fünf- oder sechsgliedrigen gesättigten Ringes erforderlichen Ringglieder bezeichnet und R für (C₁-C₄)Alkyl steht, in einer Stufe zum Endprodukt umgesetzt wird. A bezeichnet insbesondere eine Kette aus 4 oder 5 Methylengruppen. Besonders bevorzugt sind solche Ketten, in denen mindenstens eine der Methylengruppen gegen ein Stickstoff-, Sauerstoff- oder Schwefel-Brückenatom ausgetauscht ist. Eine besonders bevorzugte Verbindung der allgemeinen Formel I ist das N-Methyl-morpholin. Die drei an der Reaktion beteiligten Komponenten werden zweckmäßig in etwa äquimolaren Mengen eingesetzt. Die Umsetzung wird vorteilhaft in einem Lösemittel, wie Ethylacetat, ausgeführt, das mit den übrigen Bestandteilen des Reaktionsgemisches nicht in unerwünschter Weise reagiert.

Nachdem bereits eine Vielzahl an basischen Reaktionsbedingungen untersucht waren und sich in keinem Fall das gewünschte Ergebnis eingestellt hatte, war es für den Fachmann überraschend, daß die einstufige Reaktion in Gegenwart von Verbindungen der allgemeinen Formel I, insbesondere N-Methyl-morpholin, so glatt abläuft und das gewünschte Produkt in guter Ausbeute zugänglich macht.

Das erfindungsgemäß hergestellte t-BOC-Maleimid eignet sich insbesondere als Monomer für Polymerisationen. Die Polymere, hauptsächlich die Mischpolymere, sind besonders geeignet für sogenannte "chemisch verstärkte" strahlungsempfindliche Gemische. Die Gemische enthalten im allgemeinen neben einem Polymer, das nach einer säurekatalysierten Abspaltung von Schutzgruppen in wäßrig-alkalischer Lösung löslich wird, noch mindestens eine Verbindung, die unter Einwirkung von aktinischer Strahlung eine Säure bildet.

Prinzipiell kann das Maleimid auch nach einer Polymerisation mit tert.-Butoxycarbonyl-Schutzgruppen versehen werden. Bei einer solchen Verfahrensweise werden erfahrungsgemäß jedoch nicht alle, sondern höchstens 70 bis 90% der Maleimid-Einheiten umgesetzt. Die Ergebnisse sind außerdem schlecht reproduzierbar, d. h. man erhält die derivatisierten Polymere unter anscheinend gleichen Bedingungen mit unterschiedlichem t-BOC-Gehalt.

Strahlungsempfindliche Gemische mit Polymeren, die neben derivatisierten auch freie Maleimid-Einheiten aufweisen, zeigen eine verringerte Lagerstabilität. Die lithographischen Eigenschaften damit hergestellter Aufzeichnungsmaterialien sind schlechter als die von Aufzeichnungsmaterialien, die mit vollständig derivatisierten Polymeren hergestellt sind. Solche Polymere lassen sich jedoch nur herstellen, wenn man bereits derivatisierte Monomere einsetzt.

### Beispiel

Es wurde eine klare Lösung hergestellt aus

| | |
|---|---|
| 100 g | Maleimid (1,03 mol) |
| 225 g | Di-tert.-butyl-dicarbonat (1,03 mol) und |
| 2000 ml | Ethylacetat |

Zu dieser Lösung wurden über einen Zeitraum von 12 min 104 g (113 ml) N-Methyl-morpholin (1,03 mol) zugetropft, wobei die Temperatur der Lösung auf maximal 5°C gehalten wurde. Bei dieser Temperatur wurde noch eine Stunde nachgerührt, dann ließ man auf Raumtemperatur erwärmen und 80 h lang bei dieser Temperatur stehen. Die Lösung war nach dieser Zeit dunkel gefärbt.

Zur Aufarbeitung wurde die Lösung in 1,5 l Ethylacetat gegossen, die daraus entstandene organische Phase viermal mit Wasser gewaschen und mit Natriumsulfat getrocknet. Das Lösemittel wurde sodann abgezogen und der verbleibende Rückstand getrocknet. Man erhielt auf diese Weise 179 g eines dunkel gefärbten, kristallinen Produkts (88 % Rohausbeute).

Das Rohprodukt wurde anschließend in etwa 4,5 l Petrolether heiß gelöst, die Lösung dreimal heiß über ein Aktivkohle/Kieselgel-Bett filtriert. Die beim Abkühlen der Lösung ausfallenden Kristalle wurden sodann abgesaugt, mit Petrolether gewaschen und zum Schluß getrocknet. Auf diese Weise erhielt man 118 g eines dünnschichtchromatographisch reinen Produkts mit einem Schmelzpunkt von 66°C. Weitere 15,3 g an reinem Produkt erhielt man aus der Mutterlauge. Dazu wurde diese eingedampft, der verbleibende Rückstand mit Isopropylether aufgenommen, die resultierende Lösung heiß über ein Aktivkohle/Kieselgel-Bett filtriert und dann abgekühlt. Die ausgefallenen Kristalle wurden abgesaugt, gewaschen und getrocknet. Die gesamte Produktmenge von 133 g entspricht einer Ausbeute von 65% der Theorie.

Das Produkt wurde durch ¹H-Kernresonanz-Spektroskopie (¹H-NMR), durch Dünnschichtchromatographie und durch Elementaranalyse charakterisiert. Die erhaltenen Analysenergebnisse stimmten mit den für das gewünschte Produkt erwarteten innerhalb der üblichen Fehlergrenzen überein.
¹H-NMR (CDCl₃), chemische Verschiebung in ppm auf der δ-Skala: 6,7 [2 H] olefinische Protonen; 1,5 [9 H] Protonen der tert.-Butyl-Gruppe.

| Elementaranalyse C₉H₁₁NO₄ (MW: 197,2) | | | |
|---|---|---|---|
| | C | H | N |
| berechnet: | 54,82% | 5,62% | 7,10% |
| gefunden: | 55,0% | 5,8% | 6,9% |

## Patentansprüche

1. Verfahren zur Herstellung von N-tert.-Butoxycarbonyl-maleimid aus Maleimid und Di-tert.-butyl-dicarbonat, dadurch gekennzeichnet, daß das Maleimid mit dem Di-tert.-butyl-dicarbonat in Gegenwart einer basischen Verbindung der allgemeinen Formel I 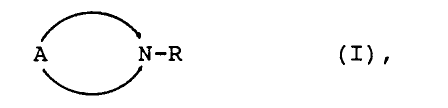 worin A die zur Vervollständigung eines fünf- oder sechsgliedrigen gesättigten Ringes erforderlichen Ringglieder bedeuten und R für (C₁-C₄)Alkyl steht,in einer Stufe zum Endprodukt umgesetzt wird.

2. Verfahren gemäß Anspruch 1, dadurch gekennzeichnet, daß A eine Kette aus 4 oder 5 Methylengruppen bezeichnet.

3. Verfahren gemäß Anspruch 2, dadurch gekennzeichnet, daß mindestens eine der Methylengruppen gegen ein Stickstoff-, Sauerstoff- oder Schwefel-Brückenatom ausgetauscht ist.

4. Verfahren gemäß Anspruch 3, dadurch gekennzeichnet, daß es sich bei der Verbindung der allgemeinen Formel I um N-Methyl-morpholin handelt.

5. Verfahren gemäß einem oder mehreren der Ansprüche 1 bis 4, dadurch gekennzeichnet, daß das Maleimid, das Di-tert.-butyl-dicarbonat und die Verbindung der allgemeinen Formel I in etwa äquimolaren Mengen eingesetzt werden.

6. Verfahren gemäß einem oder mehreren der Ansprüche 1 bis 5, dadurch gekennzeichnet, daß die Umsetzung in einem Lösemittel ausgeführt wird, das mit den übrigen Bestandteilen des Reaktionsgemisches nicht in unerwünschter Weise reagiert.

7. Verfahren gemäß Anspruch 6, dadurch gekennzeichnet, daß, daß als Lösemittel Ethylacetat verwendet wird.
